(19) Europäisches Patentamt
European Patent Office
Office européen des brevets

(11) EP 1 226 194 B1

(12) EUROPEAN PATENT SPECIFICATION

(45) Date of publication and mention of the grant of the patent:
**24.03.2004 Bulletin 2004/13**

(21) Application number: **00969747.5**

(22) Date of filing: **12.10.2000**

(51) Int Cl.$^7$: **C08F 234/02**, C07D 309/30

(86) International application number:
**PCT/IB2000/001574**

(87) International publication number:
**WO 2001/032728 (10.05.2001 Gazette 2001/19)**

(54) **SYNTHESIS OF COPOLYMERS CONTAINING ANHYDROFRUCTOSE DERIVATIVES**
SYNTHESE VON COPOLYMEREN MIT ANHYDROFRUKTOSE DERIVATEN
SYNTHESE DE COPOLYMERES CONTENANT DES DERIVES D'ANHYDROFRUCTOSE

(84) Designated Contracting States:
**AT BE CH CY DE DK ES FI FR GB GR IE IT LI LU MC NL PT SE**
Designated Extension States:
**AL LT LV MK RO SI**

(30) Priority: **04.11.1999 GB 9926175**
**27.01.2000 GB 0001939**

(43) Date of publication of application:
**31.07.2002 Bulletin 2002/31**

(73) Proprietor: **DANISCO A/S**
**1001 Copenhagen K. (DK)**

(72) Inventors:
- **GLÜMER, Anke**
**38100 Braunschweig (DE)**
- **BUCHHOLZ, Klaus**
**D-38124 Braunschweig (DE)**
- **YU, Shukun**
**S-212 40 Malmoe (SE)**

(74) Representative: **Alcock, David et al**
**D Young & Co,**
**21 New Fetter Lane**
**London EC4A 1DA (GB)**

(56) References cited:
**WO-A-99/00436**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to copolymers of anhydrofructose derivatives and at least one further monomer unit. In particular, the present invention relates copolymers of 3,6-di-O-acetyl-1,5-anhydro-4-deoxy-D-glycerohex-3-enopyranose-2-ulose, an acylated derivative of 1,5-Anhydro-D-fructose and vinylacetate, or vinylbutylether.

Polymers, including homopolymers and copolymers are extensively used in industry. Typically, the polymers are based on petroleum products and derivatives. An example of such a well know polymer is polystyrene. The synthesis of polystyrene is illustrated in Scheme 1 of Figure 1.

Petroleum based polymers are typically not biodegradable or biocompatible. As such the use thereof has become less acceptable to consumers at least and biodegradable or biocompatible alternatives have been sought.

The prior discloses some attempts to synthesise biopolymers. For example US-A-5618933 and US-A-5854030 disclose the process of Scheme 2 wherein a sugar such a glucose is enzymatically derivatised to carry a polymerisable group. The polymerisable group is then polymerised to provide a homopolymer. These processes attempt to synthesise a well-defined semi-biopolymer. In these syntheses, in place of the phenyl group as the pendant group of Scheme 1, the pendant group in these semi-biopolymers are sugar residues. US-A-5618933 and US-A-5854030 report that the presence of the sugar residues results in the polymer being a hydrogel. It is taught that the hydrogel can absorb water up to 1100 times of its own weight.

The prior art also provides teachings of polymers comprising unsaturated sugars in the backbone of the polymer (see Buchholz *et al.,* 1994. Synthesis of new "Saccharide polymers" from unsaturated monosaccharides. In: Carbohydrates as Organic Raw Materials III (edited by van Bekkum H., Röper H., and A.G.J. Voragen). VCH Publishers, Inc., New York (USA), ISBN 3-527-30079-1 AND WO- A-99/00436). However, a problem with these syntheses and the disclosed polymers has been the high cost of the unsaturated sugars incorporated in the polymer. For example the preparation of an unsaturated glucose derivative requires a multi-step process. As a result of this, the price of the unsaturated sugars and therefore the cost of the polymers prepared with the sugar derivatives are high.

The present invention addresses the problems of the prior art.

In a first aspect the present invention provides a composition comprising at least two different polymerisable monomers, (i) the first monomer unit is a polymerisable derivative of anhydrofructose; and (ii) the second monomer unit is other than a polymerisable derivative of anhydrofructose.

Preferably at least one ring of the polymerisable derivative of anhydrofructose is unsaturated.

More preferably the polymerisable derivative of anhydrofructose is of General Formula A

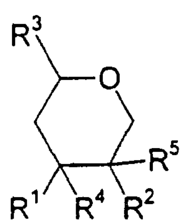

Formula A

or a derivative thereof
wherein $R^1$ and $R^2$ are independently selected from -OH, =O
wherein $R^3$ is a substituent comprising an -OH group; and
wherein $R^4$ and $R^5$ are independently selected from -OH, =O or represent a bond with an adjacent atom on the ring of the cyclic compound, wherein at least one of $R^4$ and $R^5$ represent a bond with an adjacent atom on the ring of the cyclic compound.

Preferably $R^3$ of General Formula A is or comprises an $-CH_2OH$ group.

In this respect, preferably the first monomer unit is selected from Ascopyrone M, Ascopyrone P, Ascopyrone $T_2$ and derivatives thereof.

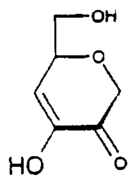

Ascopyrone M

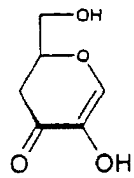

Ascopyrone P

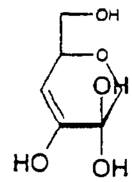

Ascopyrone $T_2$

Preferably the first monomer unit is protected. Preferably, the first monomer unit is protected by an acyl group or a benzoyl group ($C_6H_5CO$-). The first monomer unit preferably comprises an acyl group. It is well understood that the term acyl means a group R-C(=O)-.

In a preferred aspect at least one ring of the polymerisable derivative of anhydrofructose is of General Formula A (more preferably is selected from Ascopyrone M, Ascopyrone P, and Ascopyrone $T_2$) and the polymerisable derivative comprises an acyl group. Thus in a preferred aspect the first monomer unit is of the General Formula B

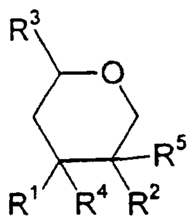

Formula B

or a derivative thereof
wherein $R^1$ and $R^2$ are independently selected from -OH, =O
wherein $R^3$ is a substituent comprising an -OH group; and
wherein $R^4$ and $R^5$ are independently selected from -OH, =O or represent a bond with an adjacent atom on the ring of the cyclic compound, wherein at least one of $R^4$ and $R^5$ represent a bond with an adjacent atom on the ring of the cyclic compound, and
wherein at least one of $R^1$ to $R^5$ is an acyl group.

More preferably the first monomer unit is of the formula

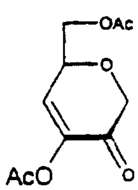

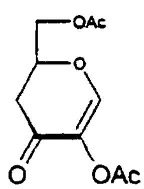

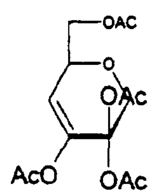

Yet more preferably, the first monomer unit is of the formula

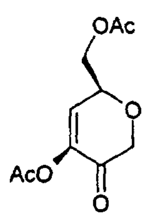

The second monomer unit may be any suitable monomer providing it is different ot the first monomer unit. Preferably the second monomer unit comprises a vinyl group. More preferably, the second monomer unit is selected from vinylacetate, vinylbutylether, styrene, derivatives and mixtures thereof.

In a further aspect the present invention provides a polymerisation product of a composition as described herein.

In a particularly preferred aspect the present invention provides a polymer comprising the unit

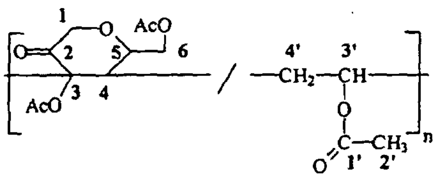

preferably

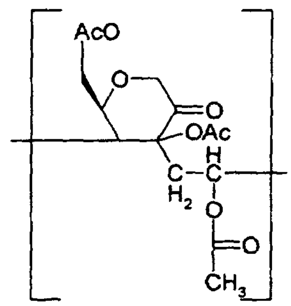

In a particularly preferred aspect the present invention provides a polymer comprising the unit

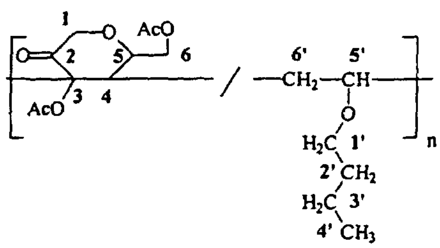

preferably

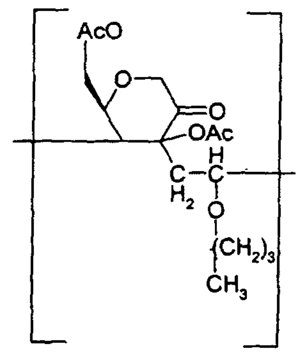

In further aspects the present invention provides a polymer comprising the unit selected from

| | | |
|---|---|---|
| 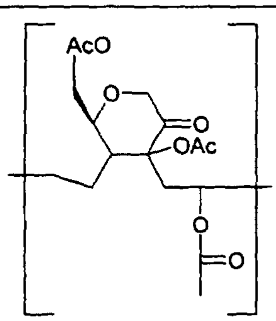 | 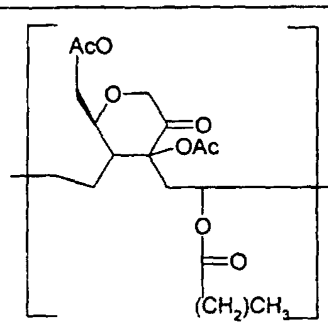 | 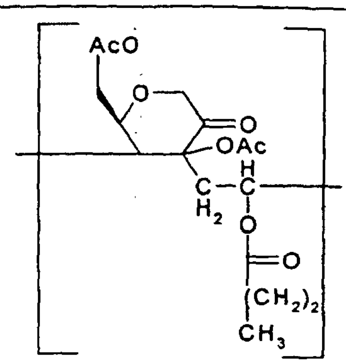 |

Anhydrofructose may be prepared for use in the present invention by any available means. In one aspect anhydrofructose may be produced directly from starch as described in S. Yu *et al.* (1999). α-1,4-Glucan lyases producing 1,5-anhydro-D-fructose from starch and glycogen have sequence similarity to alpha-glucosidases. Biochim. Biophys. Acta. 1433(1-2):1-15.

When the first monomer unit is 3,6-Di-O-acetyl-1,5-anhydro-4-deoxy-D-glycerohex-3-enopyranose-2-ulose (3,6-acetylated ascopyrone M), the monomer may be prepared from anhydrofructose according to Freimund, S. and Köpper S. 1998. Dimeric structures of 1,5-anhydro-D-fructose. Carbohydr. Res. 308: 195-200, or Andersen, S.M. *et al.* Structure of 1,5-anhydro-D-fructose: X-ray analysis of crystalline acetylated dimeric forms. J. Carbohydr. Chem. 17(7):1027-1035, 1998.

In a preferred aspect of the present invention a polymer may then be provided by the copolymerisation of 3,6-acetylated ascopyrone M with a co-monomer, such as vinylacetate, vinylbutylether. The copolymers yielded by this polymerisation are illustrated in Scheme 3. The novel sugar-based copolymers provided are Copolymer I and Copolymer II, respectively.

The polymerisation of the present invention may be carried out using polymerisation conditions well known to a person skilled in the art. The method used to prepare Copolymer I and Copolymer II is that described by Buchholz *et al.,* and WO- A-When the first monomer unit of the composition of the present invention comprises an acyl group, the acyl group may be hydrolysed after polymerisation of the composition. In this aspect, it is possible to modify the hydrophobicity of the polymer by this hydrolysis. For example the acetyl groups on Copolymer I and Copolymer II may be hydrolysed to form a series of copolymers with different degree of acetylation and therefore different degree of hydrophobicity.

The polymers of the present invention may be used in any application where provision of a hydrogel is required. For example the polymer may be used in absorbent products such as nappies (diapers) as well as in packaging materials, drug delivery polymers, medical devices such as ophthalmic devices and in a variety of other commercial applications. The present polymers may be particularly advantageous as biocompatible polymers are provided. Such polymers may be used for the preparation of topically applied materials such as cosmetics, dressings or pharmaceutical compositions which do not irritate the skin.

## EXAMPLES

Four copolymerisations were performed. Acetylated anhydrofructose derivative (AnF) was copolymerised with each of vinylacetate (Vac) and vinylbutylether (VBE). Each polymerisation was carried out in solution and in substance

EXAMPLE 1

Preparation of Poly(acetyl-Anhydro-fructose)-*co*-(Vinylacetat)

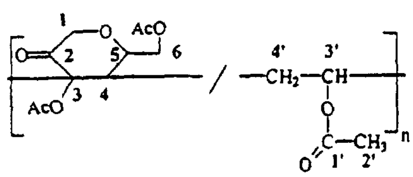

0.5 g (0.00218 mol) 3,6-Di-*O*-acetyl-1,5-anhydro-4-deoxy-D-*glycero*-hex-3-enos-2-ulopyranose was placed in a pressure stable polymerisation reactor which was equipped with a thermometer, heating, dosing device and argon inlet and outlet.

To this liquid syrup were mixed 0.242 mL (0.226 g) vinylacetate (Saccharide: Vinylacetate (1:1)) and 8 mg dibenzoylperoxide. The mixture was degassed by the freeze-thaw method. The mixture was polymerised at 80°C in the sealed reactor for 48 hours. After the reaction has finished a pale yellow solid with 14wt.% yield was obtained. The analysis by GPC-MALLS gave a weight average molecular weight of 210000 g/mol.

$^1$H-NMR (400.1 MHz; $CDCl_3$): δ = 1.6-2.3 (11H, 9H-acetyl and H-4'); 2.4-3.2 (1H, perhaps H-4); 3.2-5.2 (6H, H-1/4/5/6 and H-3').

$^{13}$C-NMR (100.6 MHz, $CDCl_3$): δ = 20-22 (2x$\underline{C}H_3$- acetyl and C-2'); 37-38 (C-4'); 52-54 (C-4); 63-80 (C-1/3/5/6 and C-3'); 165-173 (-$\underline{C}$OO- acetyl and C-1'); 200 (C-2).

FT-IR (KBr): $\tilde{\nu}$ (cm$^{-1}$) = 2962 ($CH_2$,$CH_3$); 1743 (C=O); 1432 (C-H, acetyl); 1234 ($CH_2$-Def., Ester); 1045 (C-O-Valenz).

$[\alpha]_D^{20}$ = -13.1 ($CHCl_3$, *c* = 0.655 g/100 mL).

| | | | |
|---|---|---|---|
| $(C_{10}H_{12}O_6)_{0.5}$ $(C_4H_6O_2)_{0.5}$ $(314.29)_n$ | calculated | C: 53.50 | H: 5.73 |
| | found | C:53.13 | H: 5.59 |

EXAMPLE 2

Example 1 was repeated in solution (toluene). Traces of polymer were detectable by TLC.

EXAMPLE 3

Preparation of Poly(acetyl-Anhydro-fructose)-co-(Vinylbutylether)

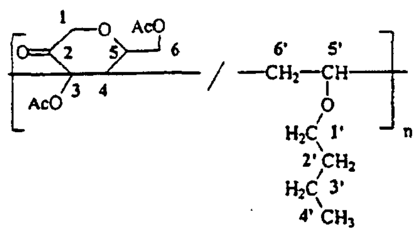

0.5 g (0.00218 mol) 3,6-Di-O-acetyl-1,5-anhydro-4-deoxy-D-glycero-hex-3-enos-2-ulopyranose was placed in a pressure stable polymerisation reactor which was equipped with a thermometer, heating, dosing device and argon inlet and outlet.

To this liquid syrup were mixed 0.281 mL (0.286 g) vinylbutylether (Saccharide: Vinylbutylether (1: 1)) and 8 mg dibenzoylperoxide and the mixture was degassed by the freeze-thaw method. The mixture was polymerised at 80°C in the sealed reactor for 48 hours. After the reaction has finished a dark yellow solid with 11wt-% yield was obtained. The analysis by GPC-MALLS gave a weight average molecular weight of 950000 g/mol.

$^1$H-NMR (400.1 MHz; $CDCl_3$): δ = 0.8-1.0 (3H, H-4'); 1.2-1.6 (4H, H-2'/3'); 1.7-2.3 (8H, $CH_3$, acetyl, H-6'); 2.4-3.7 (4H, H-1'/5' and H-4); 3.8-5.1 (5H, H-1/5/6).
$^{13}$C-NMR (100.6 MHz, $CDCl_3$): δ = 14.0 (C-4'); 19 (C-3'); 21 (C-acetyl); 32 (C-2'); 39 C-6'); 41 (C-4); 45 (C-1'); 50-52 (C-5'); 65 (C-6); 70-80 (C-1/3/5/6); 168-171 (-COO-, acetyl); 200 (C-2).
FT-IR (KBr): $\tilde{\nu}$ ($cm^{-1}$) = 2962; 2938; 2875 ($CH_2$,$CH_3$); 1745 (C=O); 1453; 1436 (C-H, acetyl); 1371;1234 ($CH_2$-Def., C-O, Ester); 1177, 1068, 1027 (C-O-Valenz, Ether).
$[\alpha]_D^{20}$ = -28.11 ($CHCl_3$, *c* = 1.11 g/100 mL).

Characterisation of

Poly(acetyl-Anhydro-fructose)-*co*-(Vinylacetat) (**AnF-Vac**) and
Poly(acetyl-Anhydro-fructose)-*co*-(Vinylbutylether) (**AnF-VBE**)

| Polymer | $\bar{M}_w$ a) (g/mol) | Tg (°C) | $[\alpha]_D^{20}$ b) (deg•$cm^2$•$dag^{-1}$) | Polymer Composition An : Comon | Yield c) (wt%) |
|---|---|---|---|---|---|
| **AnF-Vac** | 2.10•$10^5$ | ~63 | -13.1 | 58 : 42 | 14 |
| **AnF-VBE** | 9.50•$10^5$ | 81.6 | -28.1 | 40.5: 59.5 | 11 |
| Polymerisation conditions: Temperature: 80°C; Reactiontime: 48 h; Concentration of Monomers were 0.00218 mol; Initiator: Dibenzoylperoxide 1 mol%. | | | | | |

a) Weight average molecular weights determined by means of GPC online with MALLS.
b) Specific optical rotations, measured in $CHCl_3$ solution.
c) Isolated products.

EXAMPLE 4

Example 4 was repeated in solution (toluene). Traces of polymer were detectable by TLC.

EXAMPLE 5

Hydrolysis of polymers - the solid polymer of the above Examples was pulverised by ball milling, dissolved in 1-2N sodium hydroxide solution and stirred for a few days; if necessary the solution was warmed to 50°C until all polymer was dissolved. The solvent was concentrated and the pH value was adjusted to 10-11. The solution was desalted by dialysis (dialysing membrane MWCO 3500). The desalted solution was freeze dried.

EXAMPLE 6

The polymer of Example 1 is incorporated in to the liner of a child's nappy. The absorption capability of the nappy is tested by dispensing 50 ml of water on the inner surface of the nappy. The water is absorbed by the polymer of the present invention and on application of pressure to the surface of the a nappy the water is not released from the polymer.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.

## Claims

1. A composition comprising at least two different polymerisable monomers,

   (i) the first monomer unit is a polymerisable derivative of anhydrofructose; and
   (ii) the second monomer unit is other than a polymerisable derivative of anhydrofructose.

2. A composition according to claim 1 wherein at least one ring of the anhydrofructose derivative is unsaturated.

3. A composition according to claim 1 or 2 wherein the polymerisable derivative of anhydrofructose is of General Formula A

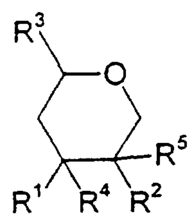

Formula A

or a derivative thereof
wherein $R^1$ and $R^2$ are independently selected from -OH, =O
wherein $R^3$ is a substituent comprising an -OH group; and
wherein $R^4$ and $R^5$ are independently selected from -OH, =O or represent a bond with an adjacent atom on the ring of the cyclic compound, wherein at least one of $R^4$ and $R^5$ represent a bond with an adjacent atom on the ring of the cyclic compound.

4. A composition according to claim 3 wherein $R^3$ is or comprises an $-CH_2OH$ group.

5. A composition according to any one of claims 1 to 3 wherein the first monomer unit is selected from Ascopyrone M, Ascopyrone P, Ascopyrone $T_2$ and derivatives thereof.

6. A composition according to any one of claims 1 to 5 wherein the first monomer unit is an acylated or benzoylated derivative of anhydrofructose.

7. A composition according to any one of claims 1 to 6 wherein the first monomer unit is of the General formula B

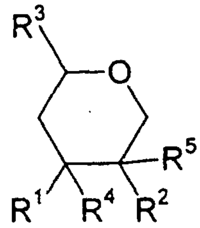

Formula B

or a derivative thereof
wherein $R^1$ and $R^2$ are independently selected from -OH, =O
wherein $R^3$ is a substituent comprising an -OH group; and
wherein $R^4$ and $R^5$ are independently selected from -OH, =O or represent a bond with an adjacent atom on the ring of the cyclic compound, wherein at least one of $R^4$ and $R^5$ represent a bond with an adjacent atom on the ring of the cyclic compound, and
wherein at least one of $R^1$ to $R^5$ is protected by an acyl group.

**8.** A composition according to any one of claims 1 to 7 wherein the first monomer unit is of the formula

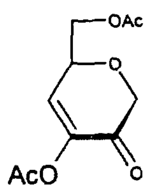
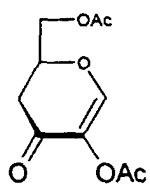
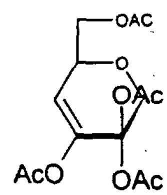

**9.** A composition according to claim 8 wherein the first monomer unit is of the formula

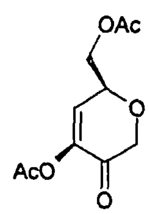

**10.** A composition according to any one of claims 1 to 9 wherein the second monomer unit comprises a vinyl group.

**11.** A composition according to any one of claims 1 to 10 wherein the second monomer unit is selected from vinylacetate, vinylbutylether, styrene, derivatives and mixtures thereof.

**12.** A polymerisation product of a composition as defined in any one of claims 1 to 11.

**13.** A polymer comprising the unit

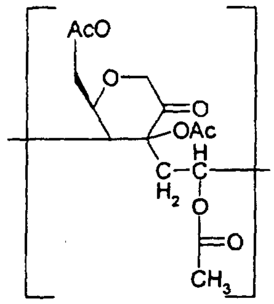

**14.** A polymer comprising the unit

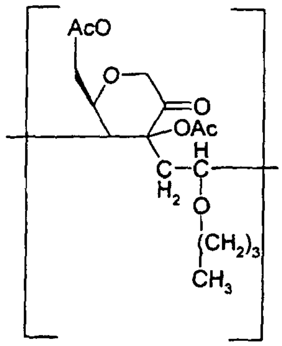

**Patentansprüche**

**1.** Zusammensetzung mit wenigstens zwei verschiedenen polymerisierbaren Monomeren, wobei

(i) die erste Monomereinheit ein polymerisierbares Derivat von Anhydrofructose und
(ii) die zweite Monomereinheit kein polymerisierbares Derivat von Anhydrofructose ist.

**2.** Zusammensetzung nach Anspruch 1, wobei wenigstens ein Ring des Anhydrofructose-Derivats ungesättigt ist.

**3.** Zusammensetzung nach Anspruch 1 oder 2, wobei das polymerisierbare Derivat von Anhydrofructose die allgemeine Formel A hat

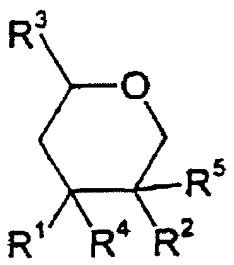

Formel A

oder ein Derivat davon ist,
wobei $R^1$ und $R^2$ unabhängig voneinander unter -OH und =O ausgewählt sind,
$R^3$ ein Substituent mit einer OH-Gruppe ist und
$R^4$ und $R^5$ unabhängig voneinander unter -OH und =O ausgewählt sind oder eine Bindung mit einem benachbarten Atom an dem Ring der zyklischen Verbindung darstellen, wobei wenigstens eines von $R^4$ und $R^5$ eine Bindung

mit einem benachbarten Atom an dem Ring der zyklischen Verbindung darstellt.

**4.** Zusammensetzung nach Anspruch 3, wobei $R^3$ eine $-CH_2OH$-Gruppe ist oder aufweist.

**5.** Zusammensetzung nach einem der Ansprüche 1 bis 3, wobei die erste Monomereinheit unter Ascopyrone M, Ascopyrone P, Ascopyrone $T_2$ und Derivaten davon ausgewählt ist.

**6.** Zusammensetzung nach einem der Ansprüche 1 bis 5, wobei die erste Monomereinheit ein azyliertes oder benzoyliertes Derivat von Anhydrofructose ist.

**7.** Zusammensetzung nach einem der Ansprüche 1 bis 6, wobei die erste Monomereinheit die allgemeine Formel B hat

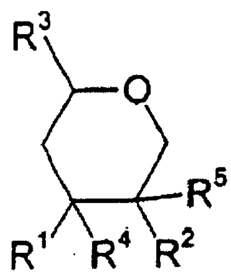

Formel B

oder ein Derivat davon ist,
wobei $R^1$ und $R^2$ unabhängig voneinander unter -OH und =O ausgewählt sind,
$R^3$ ein Substituent mit einer OH-Gruppe ist und
$R^4$ und $R^5$ unabhängig voneinander unter -OH und =O ausgewählt sind oder eine Bindung mit einem benachbarten Atom an dem Ring der zyklischen Verbindung darstellen, wobei wenigstens eines von $R^4$ und $R^5$ eine Bindung mit einem benachbarten Atom an dem Ring der zyklischen Verbindung darstellt und wobei wenigstens eines von $R^1$ bis $R^5$ durch eine Azylgruppe geschützt ist.

**8.** Zusammensetzung nach einem der Ansprüche 1 bis 7, wobei die erste Monomereinheit die folgende Formel hat:

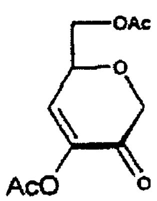
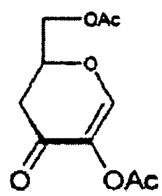
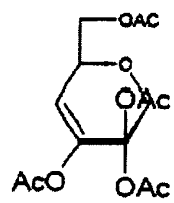

**9.** Zusammensetzung nach Anspruch 8, wobei die erste Monomereinheit die folgende Formel hat:

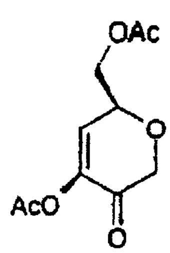

**10.** Zusammensetzung nach einem der Ansprüche 1 bis 9, wobei die zweite Monomereinheit eine Vinylgruppe aufweist.

**11.** Zusammensetzung nach einem der Ansprüche 1 bis 10, wobei die zweite Monomereinheit unter Vinylacetat, Vinylbutylether, Styren, Derivaten und Gemischen davon ausgewählt ist.

**12.** Polymerisationsprodukt einer Zusammensetzung, wie sie in einem der Ansprüche 1 bis 11 definiert ist.

**13.** Polymer, welches die folgende Einheit enthält:

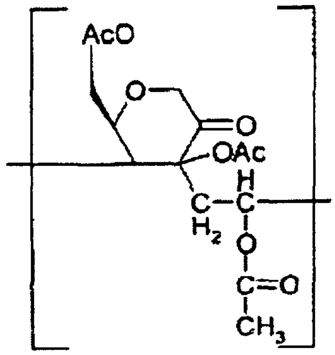

**14.** Polymer, welches die folgende Einheit enthält:

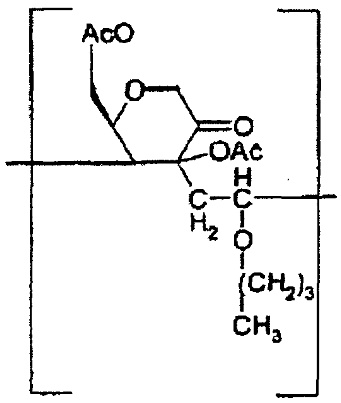

**Revendications**

**1.** Composition comprenant au moins deux monomères polymérisables différents,

(i) le premier motif monomère étant un dérivé polymérisable d'anhydrofructose ; et
(ii) le second motif monomère étant autre qu'un dérivé polymérisable d'anhydrofructose.

**2.** Composition suivant la revendication 1, dans laquelle au moins un noyau du dérivé d'anhydrofructose est insaturé.

**3.** Composition suivant la revendication 1 ou 2, dans laquelle le dérivé polymérisable d'anhydrofructose répond à la formule générale A

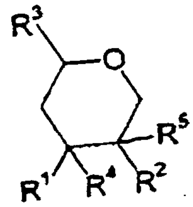

Formule A

ou un de ses dérivés,
dans laquelle $R^1$ et $R^2$ sont choisis indépendamment entre des groupes -OH, =O
dans laquelle $R^3$ est un substituant comprenant un groupe - OH ; et
dans laquelle $R^4$ et $R^5$ sont choisis indépendamment entre des groupes -OH, =O ou représentent une liaison avec un atome adjacent sur le noyau du composé cyclique, au moins un des groupes $R^4$ et $R^5$ représente une liaison avec un atome adjacent sur le noyau du composé cyclique.

**4.** Composition suivant la revendication 3, dans laquelle $R^3$ représente ou comprend un groupe $-CH_2OH$.

**5.** Composition suivant l'une quelconque des revendications 1 à 3, dans laquelle le premier motif monomère est choisi entre l'Ascopyrone M, l'Ascopyrone P, l'Ascopyrone $T_2$ et leurs dérivés.

**6.** Composition suivant l'une quelconque des revendications 1 à 5, dans laquelle le premier motif monomère est un dérivé acylé ou benzoylé d'anhydrofructose.

**7.** Composition suivant l'une quelconque des revendications 1 à 6, dans laquelle le premier motif monomère répond à la formule générale B

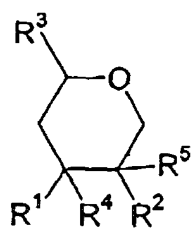

Formule B

ou un de ses dérivés,
dans laquelle $R^1$ et $R^2$ sont choisis indépendamment entre des groupes -OH, =O
dans laquelle $R^3$ représente un substituant comprenant un groupe -OH ; et
dans laquelle $R^4$ et $R^5$ sont choisis indépendamment entre des groupes -OH, =O ou représentent une liaison avec un atome adjacent sur le noyau du composé cyclique, au moins un des groupes $R^4$ et $R^5$ représente une liaison avec un atome adjacent sur le noyau du composé cyclique, et
dans laquelle au moins un des groupes $R^1$ à $R^5$ est protégé par un groupe acyle.

**8.** Composition suivant l'une quelconque des revendications 1 à 7, dans laquelle le premier motif monomère réponde à la formule

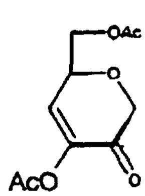

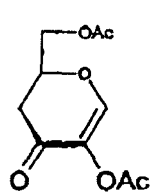

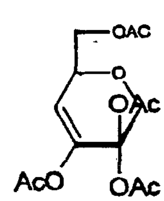

**9.** Composition suivant la revendication 8, dans laquelle le premier motif monomère répond à la formule

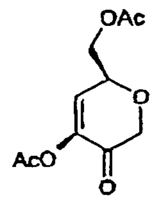

**10.** Composition suivant l'une quelconque des revendications 1 à 9, dans laquelle le second motif monomère comprend un groupe vinyle.

**11.** Composition suivant l'une quelconque des revendications 1 à 10, dans laquelle le second motif monomère est choisi entre l'acétate de vinyle, le vinylbutyléther, le styrène, leurs dérivés et leurs mélanges.

**12.** Produit polymérisable d'une composition suivant l'une quelconque des revendications 1 à 11.

**13.** Polymère comprenant le motif

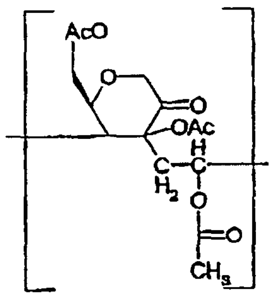

**14.** Polymère comprenant le motif

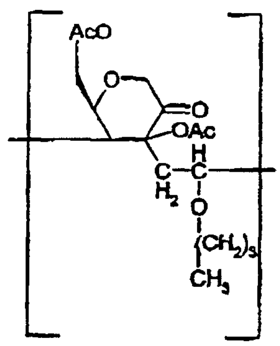

Scheme 1. Petroleum-based polymer

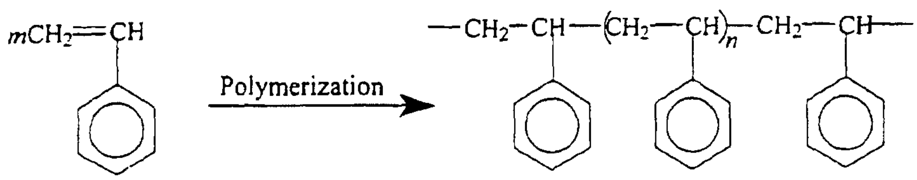


Styrene

Polystyrene

Scheme 2. Sugar-based polymers where the sugar units are hanging on the backbone

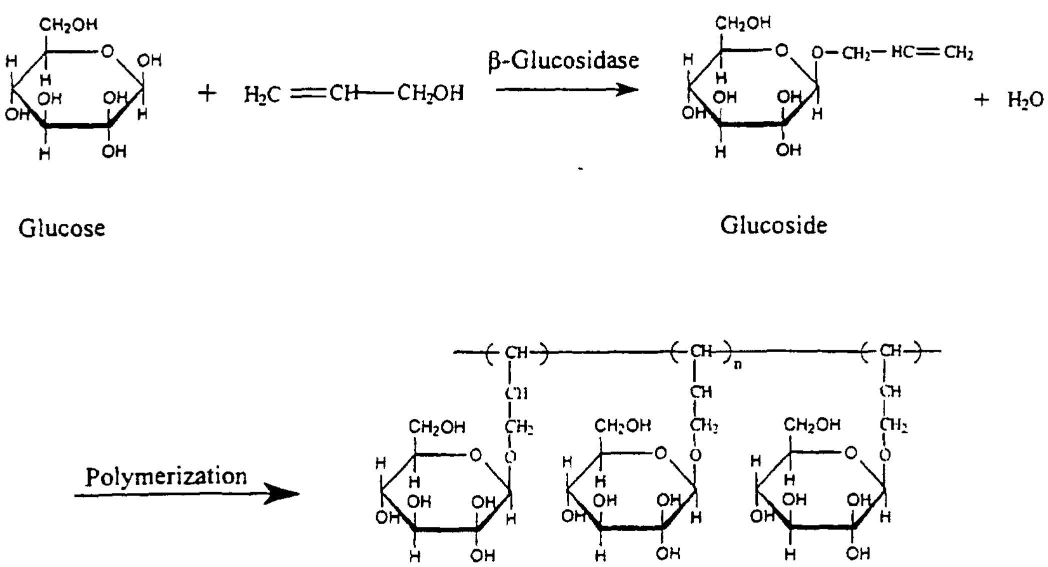


Glucose

Glucoside

Sugar-based polymer

Figure 1

## Scheme 3. Sugar-based polymers where sugar molecules are not in the backbone

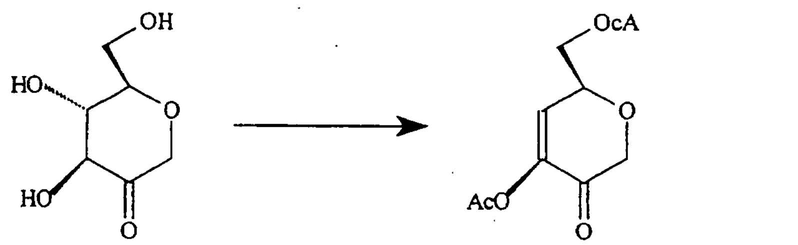

1, Anhydrofructose    2, 3,6-acetylated ascopyrone M

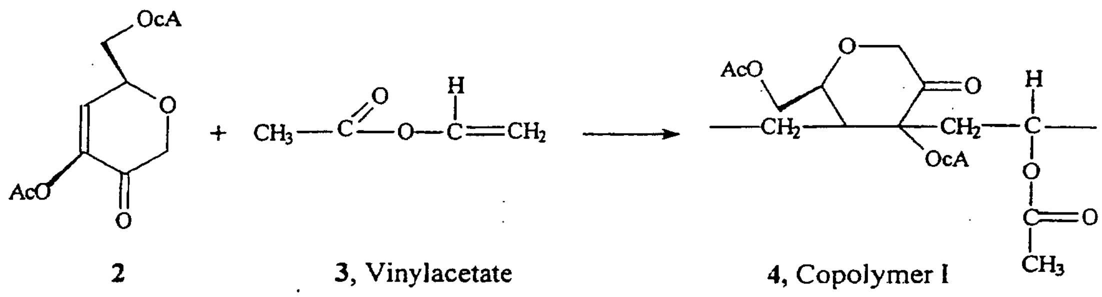

2    3, Vinylacetate    4, Copolymer I

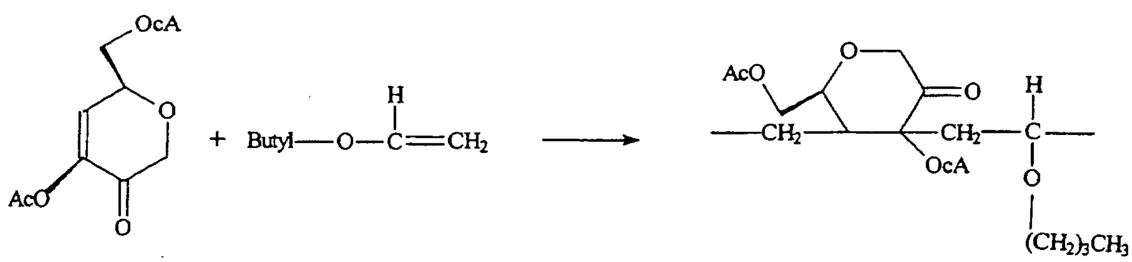

2    5, Vinylbutylether    6, Copolymer II

**Figure 2**